Europäisches Patentamt

(19) European Patent Office

Office européen des brevets

(11) Veröffentlichungsnummer: **0 070 046**
**B1**

(12) # EUROPÄISCHE PATENTSCHRIFT

(45) Veröffentlichungstag der Patentschrift:
31.07.85

(51) Int. Cl.⁴: **C 07 D 213/89**

(21) Anmeldenummer: **82200464.4**

(22) Anmeldetag: **16.04.82**

(54) Verfahren zur Herstellung des Zinksalzes von 2-Merkaptopyridin-N-oxid in einer leicht filtrierbaren aber gut suspendierbaren Form.

(30) Priorität: 15.07.81 DE 3127863

(43) Veröffentlichungstag der Anmeldung:
19.01.83 Patentblatt 83/3

(45) Bekanntmachung des Hinweises auf die Patenterteilung:
31.07.85 Patentblatt 85/31

(84) Benannte Vertragsstaaten:
BE CH DE FR GB IT LI LU NL SE

(56) Entgegenhaltungen:
EP - A - 0 034 385
US - A - 2 809 971
US - A - 3 159 640
US - A - 3 700 676

Die Akte enthält technische Angaben, die nach dem
Eingang der Anmeldung eingereicht wurden und die
nicht in dieser Patentschrift enthalten sind.

(73) Patentinhaber: Rütgerswerke Aktiengesellschaft,
Mainzer Landstrasse 217, D-6000 Frankfurt
a.Main 1 (DE)

(72) Erfinder: Maurer, Manfred, Dr., Bissersheimer
Strasse 23, D-6719 Kirchheim/Weinstr. (DE)
Erfinder: Orth, Winfried, Dr., Am Schachtelgraben 28,
D-6733 Hassloch/Pfalz (DE)
Erfinder: Fickert, Werner, Dr., Stockacher Strasse 14,
D-6800 Mannheim 61 (DE)

## Beschreibung

Die Erfindung betrifft ein Verfahren zur Herstellung des Zinksalzes von 2-Merkaptopyridin-N-oxid in einer Form, in der das Produkt leicht filtrierbar ist, sich aber dennoch gut suspendieren läßt.

Das Zinksalz des 2-Merkaptopyridin-N-oxids ist unter der Bezeichnung Zinkpyrion® ein bekanntes Handelsprodukt. Die Verbindung hat ein weites Wirkungsspektrum gegen grampositive und gramnegative Bakterien. Darüber hinaus ist sie äußerst aktiv gegen Pilze und Hefen. Die Wirksamkeit des Zink-2-merkaptopyridin-N-oxids ist pH-unabhängig. Das Produkt wird vor allem im kosmetischen Bereich als Antischuppenmittel bei der Herstellung von Shampoos eingesetzt.

Im technischen Bereich gelangen Suspensionen dieses Zinksalzes in solchen Gebieten zum Einsatz, bei denen Schutz von Angriffen durch Bakterien, Pilze und Hefen geboten ist, z. B. zur Lagerkonservierung von Dispersionsfarben und -lacken, Acrylaten oder Styrol-Butadienpolymerisaten etc.

Die Herstellung des Zinksalzes von 2-Merkaptopyridin-N-oxid ist aus US-A-2 809 971 bekannt. Man erhält es durch Umsatz wäßriger Zinksalzlösungen (z. B. Zinkchlorid, Zinksulfat) mit wäßrigen Lösungen des Natriumsalzes von 2-Merkaptopyridin-N-oxid oder mit alkoholischen Lösungen von 2-Merkaptopyridin-N-oxid.

Das auf diese Weise hergestellte Zink-2-merkaptopyridin-N-oxid hat eine durchschnittliche Korngröße von 2—3 µm und besteht in der Hauptsache aus würfel- und oktaederförmigen Kristallen, die teilweise miteinander verbacken sind. Solche feinkristallinen Niederschläge lassen sich nur sehr schwer und zeitraubend durch Filtration isolieren. Das Auswaschen von Fremdionen (z. B. $Na^+Cl^-$) gelingt nur mühsam und unter großem Zeitaufwand. Die schließlich entstehenden Pasten enthalten noch bis zu 45—48% Wasser.

Das Produkt kommt vor allem als wäßrige, weiß-cremige Suspension in den Handel. Die Suspension erhält man nach einer älteren Anmeldung durch Einwirkung von Scherkräften auf wasserhaltiges Zinksalz von 2-Merkaptopyridin-N-oxid in Gegenwart von anionischen Tensiden (Suspension mit 48% Wirkstoffgehalt) oder nichtionogenen wasserlöslichen Cellulosederivaten (Suspension mit 40% Wirkstoffgehalt). Die Stabilität solcher Suspensionen beträgt trotz der kleinen Kristallgröße maximal 2,5—3 Monate.

Aufgrund dieser Nachteile bestand demnach das dringende Bedürfnis und die Aufgabe, das Zinksalz von 2-Merkaptopyridin-N-oxid in einer Form bereitzustellen, in der es leicht isolierbar ist und sich trotzdem zu stabilen Suspensionen weiterverarbeiten läßt bzw. ein Herstellungsverfahren zu finden, in der das Zinksalz in dieser Form entsteht.

Die Aufgabe wird gelöst durch ein Verfahren zur Herstellung des Zinksalzes von 2-Merkaptopyridin-N-oxid in einer leicht filtrierbaren aber gut suspendierbaren Form, durch Umsetzen wäßriger Zinksalzlösungen mit wäßrigen Lösungen des Natriumsalzes von 2-Merkaptopyridin-N-oxid, dadurch gekennzeichnet, daß die Fällung des Zink-2-merkaptopyridin-N-oxids im Temperaturbereich von 35—100° C in Gegenwart von 10—50 Gew.-%, bezogen auf die Menge der Natriumsalzlösung, wasserlöslicher oder teilweise wasserlöslicher organischer Lösungsmittel wie niedrigeren primären, sekundären oder tertiären einwertigen Alkoholen, mehrwertigen Alkoholen, Amiden und substituierten Amiden, tertiären Aminen, Ketonen, Nitrilen, Dimethylsulfoxid, Äther, Stoffen mit gemischter Alkohol/Äther-Funktion oder Gemischen dieser Lösungsmittel erfolgt und die isolierten Kristalle gegebenenfalls mechanisch zerkleinert werden.

Aus theoretischen Überlegungen ist zu ersehen, daß sich die Filtrierbarkeit des Produktes verbessern läßt, wenn man die sich bei der Fällung bildenden Kristalle vergrößert bzw. verhindert, daß die einzelnen Kristallite miteinander verwachsen. Alle Versuche, dies über die üblicherweise angewandten Methoden wie z. B. Erhöhung der Fällungstemperatur, fraktionierte Fällung oder stärkeres Rühren zu erzielen, führten nicht zu dem gewünschten Erfolg.

Überraschenderweise wurde nun gefunden, daß beim Zusatz wasser- oder teilweise wasserlöslicher organischer Lösungsmittel, in denen das Zinksalz des 2-Merkaptopyridin-N-oxids selbst nur wenig löslich ist, zu den wäßrigen Lösungen des Natriumsalzes des 2-Merkaptopyridin-N-oxids beim Umsatz oberhalb 50° C mit wasserlöslichen Zinksalzen (z. B. Zinkchlorid oder Zinksulfat) ein kristallines Zink-2-merkaptopyridin-N-oxid mit größeren Kristalldurchmessern erhalten wird.

Als organische Lösungsmittel kommen infrage:

1. niedere, einwertige Alkohole, z. B. Methanol, Ethanol, Isopropanol, t-Butanol etc.
2. mehrwertige Alkohole, z. B. Ethylenglykol, Propylenglykol, Glycerin etc.
3. Amide und substituierte Amide, z. B. Formamid, N,N-Dimethylformamid, N,N-Diethylformamid, N,N-Diethylacetamid, Hexamethylphosphorsäuretriamid, N-Formylpyrrolidin oder -piperidin
4. tertiäre Amine, z. B. N-Methylpyrrolidin, N-Methylpiperidin
5. Ketone, z. B. Aceton, Methylethylketon
6. Nitrile, z. B. Acetonitril
7. Äther, z. B. Tetrahydrofuran, Dioxan, Ethylenglykoldimethyl- und -diethyläther

8. Stoffe mit gemischter Alkohol-Ätherfunktion, z. B. Ethylenglykolmonomethyläther, -monoethyl-äther
9. Dimethylsulfoxid
10. Gemische dieser Lösungsmittel

Besondere Vorteile bieten solche Lösungsmittel, deren Siedepunkte unter dem des Wassers liegen und die sich destillativ auf einfache Weise daraus abtrennen lassen, wie z. B. Methanol, Ethanol, Isopropanol, Aceton und Ethylenglykoldimethyläther. Diese zurückgewonnenen Lösungsmittel können wieder eingesetzt werden (unter Berücksichtigung ihres Wassergehaltes) und machen das Verfahren dadurch besonders wirtschaftlich.

Die Reaktionstemperaturen liegen im Bereich von 35—100°C, bevorzugt zwischen 60 und 70°C, wobei bei höherer Temperatur die Kristallgröße, d. h. die mittlere Länge der Längsachsen der Kristalle, des Zinksalzes des 2-Merkaptopyridin-N-oxids zunimmt.

Außerdem ist die Kristallgröße abhängig von der zugesetzten Lösungsmittelmenge. Ausgehend von Lösungen mit etwa 17 Gew.-% Natrium-2-merkaptopyridin-N-oxidlösungen genügt es, 13 Gew.-% Lösungsmittel, bezogen auf die Gewichtsmenge der Natriumsalzlösung zuzusetzen, um 5—7 µm große Kristalle zu bekommen. Eine Erhöhung der zugesetzten Lösungsmittelmenge führt zu immer größeren Kristallen. Bei einem Zusatz von 50% Lösungsmittel kann man Kristalle erhalten, die zum Teil größer als 1/2 mm sind. Das Lösungsmittel kann in seiner Gesamtmenge der wäßrigen Lösung des Natrium-2-merkaptopyridin-N-oxids oder auch teilweise dem Fällungsmittel (z. B. wäßrige, saure Zinkchloridlösung) zugegeben werden. Im Falle des Ethylenglykoldimethyläthers (13%, bei einem Verhältnis 2/3 des Äthers in der Natriumsalzlösung und 1/3 in der Fällungslösung besonders einheitlich ausgebildete Zinksalzkristalle von 5—6 µm Größe.

Es ist daher ein weiterer unerwarteter Vorteil des erfindungsgemäßen Verfahrens, daß sich neben einer großen Einheitlichkeit der Kristalle die Kristallgröße des Zinksalzes durch geschickte Kombination von Reaktionstemperatur und Lösungsmittelmenge im Bereich von 5 bis 500 µm nahezu beliebig variieren läßt.

Die entstehenden Kristalle sind überraschenderweise fast nicht miteinander verwachsen. Dadurch läßt sich der Kristallbrei leicht filtrieren und waschen, d. h. ein weiterer Vorteil liegt in der deutlich einfacheren und schnelleren Isolierbarkeit des nach der neuen Methode hergestellten Zinksalzes des 2-Merkaptopyridin-N-oxids. Bei einer Korngröße von 5—7 µm beträgt der Zeitbedarf ca. ein Drittel für die Filtration (einschließlich Waschen mit Wasser) gegenüber dem alten Verfahren. Bei einer Korngröße von etwa 100 µm beträgt es nur noch ungefähr den sechsten Teil. Darüberhinaus fällt der Wassergehalt nach dem neuen Verfahren auf durchschnittlich 20—25% gegenüber 45% nach dem alten Verfahren. Dies bringt wesentliche Vorteile beim Umgang und eine erhebliche Kostenersparnis beim Versand oder beim Trocknen der Paste.

Wenn man nun analog der eingangs geschilderten Methode durch Einwirkung von Scherkräften auf entsprechende Formulierungen, die nach dem erfindungsgemäßen Verfahren hergestelltes, feinkristallines Zinksalz des 2-Merkaptopyridin-N-oxids enthalten, anionische oder nichtionogene Suspensionen herstellt, erhält man überraschenderweise besonders stabile Produkte, deren Standzeit mehr als 5—6 Monate beträgt, ohne daß sich ein zu starker Bodenkörper absetzt. Dieser Vorteil ist völlig unerwartet, denn aufgrund der größeren Korngröße der Kristalle gegenüber denen, die aus rein wäßriger Lösung gefällt werden, war eher auf das Gegenteil zu schließen.

Eine zusätzliche Verbesserung der Stabilität von Suspensionen, die das nach dem erfindungsgemäßen Verfahren hergestellte Zinksalz enthalten, läßt sich erreichen, wenn man Zinksalz-Kristalle in der Größe von 10—200 µm bevorzugt von 50—100 µm fällt, diese mechanisch auf eine Größe von 2—8 µm zerkleinert und diese Kristalle zu einer Suspenion weiter verarbeitet. Eine derart hergestellte Suspension ist wesentlich standfester als eine Suspenion aus Zink-2-merkaptopyridin-N-oxid-Kristallen, die in einer Größe von 2—8 µm gefällt worden sind, d. h. eine derartige Suspenion ist wesentlich stabiler und der Wirkstoff setzt sich in weit geringerem Maße ab.

Es ist einleuchtend, daß diese Stabilität der Suspension umso größer ist, je weiter die Kristalle zerkleinert sind, d. h. im Prinzip sind möglichst kleine Kristalle erwünscht. Die obere Kristallgröße sollte bei etwa 8 µm liegen. Moderne Mahlgeräte, u. a. die Perl-, Sand- oder Vibrationsmühlen ermöglichen andererseits eine Zerkleinerung bis zu etwa 0,5 µm. Aus diesen Kristalltrümmern, denen keine bestimmte Kristallstruktur zuzuordnen ist, die aber eine sehr große spezifische Oberfläche haben, lassen sich außerordentlich stabile Suspensionen herstellen.

Folgende Beispiele sollen das Verfahren erläutern, ohne es zu beschränken:

## Beispiel 1

| g | ml | Mol | |
|---|----|-----|---|
| 3360 | | 4 | einer 17,7gew.-%igen Lösung des Natriumsalzes von 2-Merkaptopyridin-N-oxid und |
| 301 | 346 | | Ethylenglykoldimethyläther werden unter Rühren auf 60° C erhitzt. Zwischen 60 und 65° C tropft man ein Gemisch aus |
| 444 | 239 | 2,12 | 65proz. Zinkchloridlösung, |
| 118 | 100 | 1,18 | konzentrierte Salzsäure und |
| 134 | 154 | | Ethylenglykoldimethyläther zu. Dann kühlt man auf Raumtemperatur, saugt ab und wäscht mit |
| | 1500 | | eisenfreiem Wasser. Gut abpressen. |

Ausbeute: 848 g (25% Wasser enthaltend)
Trockenausbeute: 635 g (100% d. Th.)
Farbe: weiß bis schwach beige
Kristallgröße: 5—7 μm
Dauer der Filtration und des Waschens: 20 Min.
Lösungsmittelzusatz: 13%, bezogen auf die Gewichtsmenge der Lösung des Natriumsalzes

## Vergleichsversuch

Man arbeitet wie bei Beispiel 1 angegeben, läßt jedoch die Ethylenglykoldimethylätherzusätze weg.

Ausbeute: 1198 g (47% Wasser enthaltend)
Trockenausbeute: 635 g (100% d. Th.)
Farbe: weiß bis beige
Kristallgröße: 2—3 μm
Dauer der Filtration und des Waschens: 1 Stunde

## Beispiel 2

Man arbeitet wie in Beispiel 1 beschrieben, jedoch bei Rückflußtemperatur des zugesetzten Ethylenglykoldimethyläthers. Dabei stellt sich im Sumpf eine Temperatur von 90—93° C ein.

Ausbeute: 747 g (15% Wasser enthaltend)
Trockenausbeute: 635 g (100% d. Th.)
Farbe: beige
Kristallgröße: 10—13 μm
Dauer der Aufarbeitung: 12—15 Minuten.

## Beispiel 3

| g | ml | Mol | |
|---|---|---|---|
| 3360 | 1586 | 4 | einer 17,1gew.-%igen Lösung von Natrium-2-merkaptopyridin-N-oxid und |
| | 1680 | | N,N-Dimethylformamid oder N,N-Diethylformamid |
| | | | oder N,N-Dimethylacetamid |
| | | | werden unter Rühren auf 70—80° C erhitzt. |
| | | | Dann tropft man ein Gemisch aus |
| 444 | 239 | 2,12 | 65prozentiger Zinkchloridlösung, |
| 118 | 100 | 1,18 | konzentrierter Salzsäure |
| | | | zu, kühlt auf |
| | | | Raumtemperatur, saugt ab und wäscht mit |
| | 1500 | | eisenfreiem Wasser. |
| | | | Danach wird gut abgepreßt. |

Ausbeute: 686,5 g (7,5% Wasser enthaltend)
Trockenausbeute: 635 g (100% d. Th.)
Farbe: beige
Kristallgröße: um 500 µm
Dauer der Aufarbeitung: knapp 10 Minuten
Lösungsmittelzusatz: 47%, bezogen auf die Gewichtsmenge der Lösung des Natriumsalzes

## Beispiel 4

Man arbeitet wie bei Beispiel 1 beschrieben, verwendet anstelle von Ethylenglykoldimethyläther aber Methanol, Ethanol, Isopropanol, t-Butanol, Aceton, Acetonitril, Dioxan oder Tetrahydrofuran.

Ausbeute: 900 g ( ~ 30% Wasser enthaltend)
Trockenausbeute: 635 g (100%)
Farbe: weiß bis beige
Kristallgröße: 3—6 µm
Dauer der Aufarbeitung: etwa 30—35 Minuten.

## Beispiel 5

Man arbeitet wie in Beispiel 2 beschrieben, anstelle von Ethylenglykoldimethyläther werden aber Ethylenglykolmonomethyläther, Ethylenglykoldiethyläther oder Formamid eingesetzt.

Ausbeute: ~980 g ( ~33% Wasser enthaltend)
Trockenausbeute: 635 g (100%)
Farbe: beige
Kristallgröße: 3—5 µm
Dauer der Aufarbeitung: 35—40 Minuten.

0 070 046

## Beispiel 6

| g | ml | Mol | |
|---|---|---|---|
| 3360 | | | einer 17,1gew.-%igen Lösung von Natrium-2-merkaptopyridin-N-oxid und |
| 512 | 500 | | Hexamethylphosphorsäuretriamid werden auf 70—80° C erhitzt. Dazu tropft man ein Gemisch aus |
| 444 | 239 | 2,12 | 65prozentiger Zinkchloridlösung, |
| 118 | 100 | 1,18 | konzentrierter Salzsäure und |
| 176 | 172 | | Hexamethylphosphorsäuretriamid zu, kühlt auf Raumtemperatur, saugt ab und wäscht mit |
| | 1500 | | eisenfreiem Wasser. Danach wird gut abgepreßt. |

Ausbeute: 722 g (12% Wasser enthaltend).
Trockenausbeute: 635 g (100% d. Th.)
Farbe: weiß — beige
Kristallgröße: um 100 μm
Lösungsmittelzusatz: 20%, bezogen auf die Gewichtsmenge der Lösung des Natriumsalzes
Dauer der Aufarbeitung: 10—15 Minuten.

## Beispiel 7

Man arbeitet nach Beispiel 1, anstelle von Ethylenglykoldimethyläther werden jedoch N-Methylpyrrolidin, N-Methylpiperidin, N-Formylpyrrolidin oder Dimethylsulfoxid verwendet.

Ausbeute: 882 g (~ 28% Wasser enthaltend)
Trockenausbeute: 635 g (100% d. Th.)
Farbe: weiß bis schwach beige
Kristallgröße: 4—6 μm
Aufarbeitungsdauer: etwa 20 Minuten.

## Beispiel 8

| g | ml | Mol | |
|---|---|---|---|
| 3360 | | 4 | einer 17,1prozentigen Lösung von Natrium-2-merkaptopyridin-N-oxid und |
| 541 | 672 | | Methylethylketon werden unter Rühren auf 70° C erhitzt. Dazu tropft man ein Gemisch aus |
| 444 | 239 | 2,12 | 65prozentiger Zinkchloridlösung und |
| 118 | 100 | 1,18 | konzentrierter Salzsäure, kühlt auf Raumtemperatur, saugt ab und wäscht mit |
| | 1500 | | eisenfreiem Wasser. Gut abpressen. |

Ausbeute: 794 g (20% Wasser enthaltend)
Trockenausbeute: 635 g (100% d. Th.)
Farbe: weiß — beige
Kristallgröße: 6—10 μm
Dauer der Aufarbeitung: etwa 20 Minuten
Lösungsmittelzusatz: 16%

## Beispiel 9

Zink-2-merkaptopyridin-N-oxid wurde in Wasser in einer Konzentration von 48 Gew.-% unter geringem Zusatz eines anionischen Tensids unter Einwirkung von Scherkräften suspendiert.
Dabei wurden folgende Formen des Zink-2-merkaptopyridin-N-oxids eingesetzt:

6

**0 070 046**

a) herkömmliche Kristallform, ohne Zugabe von Lösungsmittel gefällt; 2—3 μm Größe (analog Vergleichsbeispiel).
b) herkömmliche Kristallform, ohne Zusatz von Lösungsmittel in einer Größe von 2—3 μm gefällt und danach auf eine Größe von 0,5—2 μm zerkleinertes Zinksalz.
c) erfindungsgemäß unter Zugabe von Lösungsmittel (analog Beispiel 1) in einer Größe von 5—7 μm gefälltes Zinksalz.
d) erfindungsgemäß unter Zugabe von Lösungsmittel (analog Beispiel 6) in einer Größe von etwa 100 μm gefälltes und danach auf eine Größe von 5—8 μm zerkleinertes Zinksalz.
e) erfindungsgemäß unter Zugabe von Lösungsmittel (analog Beispiel 6) in einer Größe von etwa 100 μm gefälltes und danach auf eine Größe von 0,5—2 μm zerkleinertes Zinksalz.

Die Suspensionen wurden jeweils in einem Mischzylinder mit 30 cm Höhe gefüllt und 6 Monate bei Raumtemperatur ruhig gelagert. Danach wurde folgendes Absetzverhalten beobachtet:

a) klare, überstehende Flüssigkeitssäule von 21 mm Höhe, starker Bodensatz, der nicht mehr aufrührbar ist.
b) klare, überstehende Flüssigkeit von 14 mm Höhe, deutlicher Bodensatz, schwer aufrührbar.
c) klare, überstehende Flüssigkeit von 11 mm Höhe, schwacher Bodensatz, gut aufrührbar.
d) leicht trübe, überstehende Flüssigkeit von 5 mm Höhe, kein Bodensatz.
e) trübe, überstehende Flüssigkeit von 2 mm Höhe, kein Bodensatz.

Beispiel 10

2,4 g Hydroxyäthylcellulose werden unter intensivem Rühren in 530 ml Wasser gelöst. In diese Lösung trägt man unter Rühren 470 g eines nach Beispiel 2 hergestellten Zink-2-merkaptopyridin-N-oxids (Wassergehalt 15 Gew.-%) ein. Die so hergestellte Suspension wird den Scherkräften eines modernen Mahlgerätes unterworfen und dabei die Zinksalzkristalle auf 0,5—2 μm zerkleinert. Man erhält eine etwa 40%ige Suspension, deren Stabilität so groß ist, daß im Absetztest gemäß Beispiel 9 nahezu kein Absetzen beobachtet wird.

**Patentansprüche**

1. Verfahren zur Herstellung des Zinksalzes von 2-Merkaptopyridin-N-oxid in einer leicht filtrierbaren aber gut suspendierbaren Form, durch Umsetzen wäßriger Zinksalzlösungen mit wäßrigen Lösungen des Natriumsalzes von 2-Merkaptopyridin-N-oxid, dadurch gekennzeichnet, daß die Fällung des Zink-2-merkaptopyridin-N-oxids im Temperaturbereich von 35—100°C in Gegenwart von 10—50 Gew.-%, bezogen auf die Menge der Natriumsalzlösung, wasserlöslicher oder teilweise wasserlöslicher organischer Lösungsmittel wie niedrigeren primären, sekundären oder tertiären einwertigen Alkoholen, mehrwertigen Alkoholen, Amiden und substituierten Amiden, tertiären Aminen, Ketonen, Nitrilen, Dimethylsulfoxid, Äther, Stoffen mit gemischter Alkohol/Äther-Funktion oder Gemischen dieser Lösungsmittel erfolgt und die isolierten Kristalle gegebenenfalls mechanisch zerkleinert werden.
2. Verfahren nach Anspruch 1, dadurch gekennzeichnet, daß die Fällungsreaktion im Temperaturbereich von 60—70°C durchgeführt wird.
3. Verfahren nach Anspruch 1 bis 2, dadurch gekennzeichnet, daß man die Lösungsmittel in Mengen von 10—15 Gew.-%, bezogen auf die Menge der Natrium-2-merkaptopyridin-N-oxid-Lösung, zusetzt.
4. Verfahren nach Anspruch 1 oder 2, dadurch gekennzeichnet, daß die Fällungsreaktion im Temperaturbereich von 60—80°C und mit einem Lösungsmittelzusatz von 20—40 Gew.-%, bezogen auf die Menge der Natrium-2-merkaptopyridin-N-oxid-Lösung, durchgeführt wird und daß die erhaltenen 10—200 μm großen Kristalle des Zink-2-merkaptopyridin-N-oxids nach der Isolierung mechanisch zerkleinert werden.
5. Verfahren nach Anspruch 4, dadurch gekennzeichnet, daß die Kristalle des Zink-2-merkaptopyridin-N-oxids auf eine durchschnittliche Größe von 2—8 μm zerkleinert werden.
6. Verfahren nach Anspruch 4, dadurch gekennzeichnet, daß die Kristalle des Zink-2-merkaptopyridin-N-oxids auf eine durchschnittliche Größe von 0,5—2 μm zerkleinert werden.
7. Verwendung der nach Ansprüchen 1—6 hergestellten Zinksalze des 2-Merkaptopyridin-N-oxids in Suspensionen.

**Claims**

1. A process for the preparation of the zinc salt of 2-mercaptopyridine-N-oxide in a readily filterable, but easily suspendable form comprising reacting an aqueous solution of an alkali metal salt of 2-mercaptopyridine-N-oxide and an aqueous solution of a water soluble zinc salt, characterized in that

7

the precipitation of the zinc salt of 2-mercaptopyridine-N-oxide is made in the temperature range of 35—100°C in the presence of 10—50 percent by weight, based on the quantity of the sodium salt solution of an water soluble or partially water soluble organic solvent selected from the group consisting of secondary or tertiary alkanols, polyvalent alcohols, amides and substituted amides, teritary amines, ketones, nitriles, dimethyl sulfoxide, ether, substances with mixes alcohol/ether function and mixtures of the said solvents, and that the isolated crystals are possibly crushed mechanically.

2. A process of claim 1, characterized in that the precipitation is made in the temperature range of 60 to 70°C.

3. A process of claims 1 to 2, characterized in that the organic solvents are added in quantities of 10 to 15 % by weight, based on the weight of the sodium 2-mercaptopyridine-N-oxide solution.

4. A process of claims 1 or 2, characterized in that the precipitation is carried out at a temperature of about 60 to 80°C; with a solvent addition of 20 to 40% by weight based on the weight of sodium 2-mercaptopyridine-N-oxide solution, to obtain 10 to 200 micron crystals of the zinc 2-mercaptopyridine-N-oxide and mechanically comminuting the crystals after isolation.

5. A process of claim 4, characterized in that the crystals of the zinc 2-mercaptopyridine-N-oxide are comminuted to an average size of 2 to 8 microns.

6. A process of claim 4, characterized in that the crystals of the zinc 2-mercaptopyridine-N-oxide are comminuted to an average size of 0,5 to 2 microns.

7. Use of the zinc salts of 2-mercaptopyridine-N-oxide of claims 1 to 6 in suspensions.

## Revendications

1. Procédé pour préparer le sel de zinc du mercapto-2 pyridine-N-oxyde sous une forme facile à filtrer mais facile à mettre en suspension, par réaction de solutions aqueuses de sels de zinc avec des solutions aqueuse du sel sodique du mercapto-2 pyridine-N-oxyde, procédé caractérisé en ce que la précipitation du sel de zinc du mercapto-2 pyridine-N-oxyde est effectuée dans un intervalle de température allant de 35 à 100°C, en présence de 10 à 50% en poids (par rapport à la quantité de la solution de sel sodique) de solvants organiques solubles dans l'eau ou partiellement solubles dans l'eau, tels que des monoalcools inférieurs, primaires, secondaires ou tertiaires, des polyols, des amides et des amides substitués, des amines tertiaires, des cétones, des nitriles, le diméthylsulfoxyde, des éthers, des substances ayant à la fois une fonction alcool et une fonction éther, ou des mélanges de ces solvants, et les cristaux isolés sont éventuellement broyés mécaniquement.

2. Procédé selon la revendication 1, caractérisé en ce que la réaction de précipitation est effectuée dans l'intervalle de température allant de 60 à 70°C.

3. Procédé selon l'une des revendications 1 ou 2, caractérisé en ce que les solvants sont ajoutés en des quantités de 10 à 15% en poids par rapport à la quantité de la solution du sel sodique du mercapto-2 pyridine-N-oxyde.

4. Procédé selon l'une quelconque des revendications 1 et 2, procédé caractérisé en ce que la réaction de précipitation est effectuée dans un intervalle de température allant de 60 à 80°C et avec une quantité de solvant ajoutée comprise entre 20 et 40% en poids par rapport à la quantité de la solution du sel sodique du mercapto-2 pyridine-N-oxyde, et en ce que les cristaux du sel de zinc du mercapto-2 pyridine-N-oxyde obtenus, qui ont des dimensions de 10 à 200 µm, sont broyés mécaniquement après avoit été isolés.

5. Procédé selon la revendication 4, caractérisé en ce qu'on broie les cristaux du sel de zinc du mercapto-2 pyridine-N-oxyde à une dimension moyenne de 2 à 8 µm.

6. Procédé selon la revendication 4, caractérisé en ce qu'on broie les cristaux du sel de zinc du mercapto-2 pyridine-N-oxyde à une dimension moyenne de 0,5 à 2 µm.

7. Application des sels de zinc du mercapto-2 pyridine-N-oxyde préparés selon l'une quelconque des revendications 1 à 6, dans des suspensions.